(19) Europäisches Patentamt
European Patent Office
Office européen des brevets

(11) Veröffentlichungsnummer: **0 000 912**
**B1**

(12) **EUROPÄISCHE PATENTSCHRIFT**

(51) Int. Cl.³: **C 07 H 3/04**, C 07 H 3/02, C 07 C 49/17, C 07 C 47/19, C 07 C 31/18, C 07 C 29/14, C 07 C 29/00, // C 08 G 18/42, C 08 G 18/48

(21) Anmeldenummer: **78100659.8**

(22) Anmeldetag: **14.08.78**

# (54) Verfahren zur Herstellung von niedermolekularen Polyhydroxylverbindungen

(30) Priorität: **26.08.77 DE 2738512**

(43) Veröffentlichungstag der Anmeldung:
**07.03.79 Patentblatt 79/05**

(45) Bekanntmachung des Hinweises auf die Patenterteilung:
**01.10.80 Patentblatt 80/20**

(84) Benannte Vertragsstaaten:
**BE CH DE FR GB LU NL**

(56) Entgegenhaltungen:
**GB - A - 513 708**

(73) Patentinhaber: **BAYER AG**
**Zentralbereich Patente, Marken und Lizenzen**
**5090 Leverkusen 1, Bayerwerk (DE)**

(72) Erfinder: **Müller, Hanns Peter, Dr.**
**Berta-von-Suttner-Strasse 40**
**D - 5090 Leverkusen 1 (DE)**
**Wagner, Kuno, Dr.**
**Am Kiesberg 8**
**D - 5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 000 912 B1

Courier Press, Leamington Spa, England.

Verfahren zur Herstellung von niedermolekularen Polyhydroxylverbindungen

Die vorliegende Erfindung betrifft ein verbessertes Verfahren zur Herstellung eines Gemisches von niedermolekularen, mehrwertigen Alkoholen, Hydroxyaldehyden und Hydroxyketonen durch Kondensation von Formaldehydhydrat. Derartige Gemische werden im folgenden als "Formose" bezeichnet.

Polyhydroxylverbindungen haben auf den verschiedensten Gebieten große technische Bedeutung erlangt. Sie werden beispielsweise für die Herstellung von nichtionischen oberflächenaktiven Verbindungen, als Gefrierschutzmittel, Feucht- und Weichhaltemittel sowie als Ausgangskomponenten für Kunststoffe, wie z.B. Polyester- und Polyätherharze großtechnisch eingesetzt.

Seit den Arbeiten von Butlerow und Loew (Ann. *120,* 295 (1861) und J. pr. Chem. *33,* 321 (1886)) im vorigen Jahrhundert ist es bekannt, daß sich bei der Selbstkondensation des Formaldehydhydrates (im folgenden soll unter "Selbstkondensation des Formaldehyds" immer "Selbstkondensation des Formaldehydhydrats" verstanden werden) unter dem Einfluß von basischen Verbindungen, wie z.B. Calciumoder Bleihydroxid, Hydroxyaldehyde und Hydroxyketone bilden.

Trotz vieler Vorschläge zur Synthese von Polyhydroxylverbindungen durch Selbstkondensation von Formaldehyd ist aber bis zur Gegenwart kein technisch brauchbares Verfahren hierfür entwickelt worden, weil es bisher nicht gelungen war, Gemische von Polyhydroxylverbindungen mit definierter Reproduzierbarkeit der Hydroxylfunktionalität zu synthetisieren. Bei den bekannten Verfahren werden außerdem Hydroxyaldehyd- und Hydroxyketongemische erhalten, die sich nur schwer und it sehr großen Katalysatormengen hydrieren lassen. Dieser hohe Katalysatorverbrauch hat die Synthese von Polyhydroxylverbindungen durch Selbstkondensation des Formaldehydhydrats bisher unwirtschaftlich erscheinen lassen und verhindert, daß die Selbstkondensation des Formaldehydhydrats als Grundlage eines technischen Verfahrens zur Synthese von mehrwertigen Alkoholen herangezogen wurde.

Infolge der gleichzeitig ablaufenden Disproportionierung des Formaldehyds zu Methanol und Ameisensäure konnten mit den bisher bekannten Verfahren meist nur mäßige Ausbeuten erzielt werden, so daß die Aufarbeitung der entstehenden wäßrigen bzw. wäßrig/alkoholischen Lösungen erhebliche wirtschaftliche Kosten verursacht.

Bekanntlich wird die Disproportionierung von Formaldehyd in Methanol und Ameisensäure durch basische Verbindungen sehr stark katalysiert. Wie Pfeil, Chemische Berichte *84.* 229 (1951) feststellte, hängt die Reaktionsgeschwindigkeit dieser sogenannten "Cannizzaro-Reaktion" vom Quadrat der Formaldehydkonzentration ab, während die Reaktionsgeschwindigkeit der Formaldehydpolyaddition (C—C-Verknüpfung) linear von der Formaldehydkonzentration abhängt (Pfeil und Schroth, Chemische Berichte *85,* 303 (1952)). Mit steigender Aldehydkonzentration wird daher das Mengenverhältnis von gewünschten Polyhydroxylverbindungen zu Methanol und Ameisensäure zu Ungunsten der gesuchten Verbindungen verschoben. Daher wird in den meisten zum Stand der Technik gehörenden Verfahren vorgeschlagen, die Kondensation des Formaldehyds zu Hydroxyaldehyden und Hydroxyketonen in Lösungen mit niedrigen Formaldehydkonzentrationen durchzuführen, um die Menge an Nebenprodukten so niedrig wie möglich zu halten. Zur Gewinnung der gebildeten Hydroxyaldehyde und Hydroxyketone ist es jedoch notwendig, das als Lösungsmittel verwendete Wasser wieder destillativ zu entfernen. Bedingt durch die hohe Verdampfungswärme des Wassers entstehen dadurch erhebliche Energiekosten. Verfahren zur Kondensation des Formaldehyds aus verdünnten wäßrigen Lösungen sind aus diesem Grunde unwirtschaftlich. Außerdem treten bei längeren Destillationszeiten in erheblichem Maß Zersetzungs- und Verfärbungsreaktionen der gebildeten Hydroxyaldehyde und Hydroxyketone auf.

Es ist daher wünschenswert, die Kondensation des Formaldehyds aus handelsüblichen konzentrierten Formalinlösungen durchzuführen, ohne daß dabei störende Nebenreaktionen auftreten.

Zur Vermeidung der Cannizzaro-Reaktion wurde weiter vorgeschlagen, Formaldehydlösungen in Gegenwart von Methanol, Äthanol oder anderen polaren organischen Lösungsmitteln mit Calcium- oder Bleihydroxid umzusetzen (Deutsche Patentschrift 830 951 sowie Gorr und Wagner, Biochemische Zeitschrift, *262,* 361 (1933)).

Durch Zugabe von organischen Lösungsmitteln wird jedoch der Formaldehydgehalt der Lösung wiederum verringert.

Die zusätzlich erforderlichen Energiekosten zur Verdampfung des zugesetzten Lösungsmittels bei der Aufarbeitung der gebildeten Hydroxyaldehyde und -ketone lassen daher auch diese Verfahren unwirtschaftlich erscheinen.

Aus der US-Patentschrift 2 224 910 ist ein Verfahren zur Herstellung von Hydroxyaldehyden und Hydroxyketonen bekannt geworden, bei dem die exotherme Selbstkondensation des Formaldehyds durch kontrollierte Zugabe von anorganischen oder organischen Basen zu einer Formaldehydlösung, die Blei-, Zinn-, Calcium-, Barium-, Magnesium-, Cer- oder Thoriumverbindungen sowie eine zur Endiolbildung befähigte Verbindung, wie Glucose, Ascorbinsäure, Fruktose, Benzoin, Glykolaldehyd, Erythrose, Reduktose, Invert-Zucker oder Kondensationsprodukte des Formaldehyds, enthält, geregelt wird. Man erhält bei diesem Verfahren zwar ein Gemisch von Hydroxyaldehyden und Hydroxyketonen aus Formaldehydlösungen höherer Konzentration ohne Zusatz von organischen Lösungsmitteln, muß jedoch verschiedene Nachteile in Kauf nehmen. So werden, wenn man die Umsetzung bei niedrigen

pH-Werten ablaufen läßt, vor allem Hydroxyaldehyd- und Hydroxyketongemische mit niedriger Hydroxyfunktionalität erhalten. Außerdem werden bei niedrigen pH-Werten nur mäßige Reaktionsgeschwindigkeiten erzielt, so daß die Raum-Zeit-Ausbeuten dieser Verfahrensvarianten nicht befriedigen. Um diese Nachteile zu umgehen, wird in der zitierten Patentschrift empfohlen, die Formaldehydkondensation bei niedrigen pH-Werten zu starten und dann bei höheren pH-Werten zu Ende zu führen. Bei pH-Werten $\geq 7$ läuft die Blei-katalysierte Formaldehydselbstkondensation aber dann so rasch, spontan und unkontrolliert ab, daß es nach dieser Verfahrensvariante nicht möglich ist, Gemische von Hydroxyaldehyden und Hydroxyketonen mit reproduzierbarer Komponentenverteilung zu erhalten, weil die Reaktionszeiten und -bedingungen nicht mehr exakt kontrollierbar sind. Es ist darüber hinaus bekannt, daß sich Hydroxyaldehyde, Hydroxyketone und Monosaccharide im alkalischen Milieu und bei erhöhter Temperatur zu dunkel gefärbten, teilweise carboxylgruppenhaltigen Verbindungen zersetzen.

Diese Zersetzungsreaktionen treten insbesondere bei den als bevorzugt vorgeschlagenen Verfahrensweisen von US 2 224 910 auf, vor allem, nachdem die Hauptmenge des Formaldehyds umgesetzt ist. Hydroxyaldehyd- und Hydroxyketongemische, wie sie nach dem Verfahren der US-Patentschrift 2 224 910 hergestellt worden sind, enthalten somit Zersetzungsprodukte mit sauren Gruppen, sind braun gefärbt und können nicht reproduzierbar hergestellt werden. Die Hydrierung dieser Gemische gelingt darüber hinaus nur mit unwirtschaftlich hohen Mengen an Raney-Nickel-Katalysator. So werden zur Hydrierung einer zu 100 g Formaldehyd äquivalenten Menge an Hydroxyaldehyd- und Hydroxyketongemisch 30 g Raney-Nickel benötigt.

Die Produktgemische, die nach der eben beschriebenen Verfahrensweise erhalten worden sind, müssen zur Reinigung und zur Gewinnung von Hydroxylverbindungen mit niedrigem Molekulargewicht in jedem Fall destillativ aufgearbeitet werden. Es wäre jedoch wünschenswert, die destillative Aufarbeitung des Gemisches, die zusätzliche Energie- und Apparatekosten verursacht, einzusparen und die Produktgemische so herzustellen, daß sie direkt nach Entfernung des Lösungswassers ohne zusätzlich Destillation weiterverwendet werden können. Solche farblosen, von Nebenprodukten weitgehend freien Reaktionsgemische sind nach den Verfahren des Standes der Technik aber nicht zu erhalten.

Der vorliegenden Erfindung liegt daher die Aufgabe zugrunde, ein Verfahren bereitzustellen, nach welchem Gemische von Polyhydroxylverbindungen synthetisiert werden können, die möglichst frei von Zersetzungsprodukten sind und die auf einfache Weise mit geringen Mengen an Hydrierkatalysatoren zu mehrwertigen Alkoholen hydriert werden können. Die erhaltenen Gemische von Polyhydroxylverbindungen sollen farblos sein und keiner weiteren Reinigung bedürfen.

Aufgabe der vorliegenden Erfindung war es ferner, die Formaldehydselbstkondensation so zu steuern, daß sich die Produktverteilung der entstehenden Gemische von niedermolekularen Polyhydroxylverbindungen je nach Anwendungswunsch variieren und reproduzierbar einstellen läßt.

Überraschenderweise wurde nun gefunden, daß sich farblose und von störenden Nebenprodukten weitgehend freie Formosen mit hohen Raum/Zeit-Ausbeuten herstellen lassen, wenn man die Kondensation des Formaldehydhydrats bei basischen pH-Werten in Gegenwart von Calciumhydroxid als Katalysator und von zur Endiolbildung befähigten Verbindungen als Cokatalysator ablaufen läßt, sofern man die Konzentration des Formaldehyds im Reaktionsgemisch durch eine geeignete Dosiergeschwindigkeit der als Formaldehydquelle eingesetzten wäßrigen Formalinlösung und/oder Paraformaldehyd-Dispersion so steuert, daß eine gewisse Mindest- bzw. Maximalmenge, bezogen auf das gesamte Reaktionsgemisch, nicht unter- bzw. überschritten wird. Es ist dabei als besonders überraschend anzusehen, daß trotz der hohen pH-Werte keine Verbräunungs- und Zersetzungsreaktionen der Formose stattfinden und daß gekreuzte Cannizzaro-Reaktionen im Vergleich zu bisher bekannten Calciumhydroxid-katalysierten Formosesynthesen stark zurückgedrängt werden, so daß die entstehenden Formosen einen relativ hohen Gehalt an reduzierenden Gruppen aufweisen.

Gegenstand der vorliegenden Erfindung ist somit ein Verfahren zur Herstellung von niedermolekularen Polyhydroxylverbindungen durch Kondensation von Formaldehydhydrat in Gegenwart von Calciumhydroxid als Katalysator sowie von zur Endiolbildung befähigten Verbindungen als Cokatalysator, welches dadurch gekennzeichnet ist, daß man eine Formaldehyd enthaltende Lösung des Cokatalysators in Wasser und gegebenenfalls neidermolekularen ein- oder mehrwertigen Alkoholen und/oder höhermolekularen Polyhydroxylverbindungen bei einer Temperatur von 80 bis 110°C, vorzugsweise 90 bis 105°C, durch Zusatz von Calciumhydroxid auf einen pH-Wert von 9 bis 12, vorzugsweise 9 bis 10, bringt, so daß die Kondensation des Formaldehydhydrats gestartet wird, anschließend eine 20 bis 65 Gew.-% Formaldehyd enthaltende wäßrige Formalinlösung und/oder Paraformaldehyd-Dispersion sowie Calciumhydroxid so zudosiert, daß das Reaktionsgemisch bei einer Temperatur von 80 bis 110°C, bevorzugt 90 bis 105°C, und in einem pH-Bereich zwischen 7,5 und 9,5, vorzugsweise zwischen 8 und 9, gehalten wird, wobei während des ganzen Verlaufs der Kondensationsreaktion die Formaldehydkonzentration zwischen 0,5 und 10 Gew.-%, bevorzugt zwischen 1,2 und 6 Gew.-%, bezogen auf gesamtes Reaktionsgemisch, gehalten wird und schließlich gegebenenfalls die Restmenge von 0,5 bis 10 Gew.-% Formaldehyd durch weitere Kondensation bei pH-Werten unter 7 oder durch Reaktion mit anderen gegenüber Formaldehydhydrat reaktiven Verbindungen entfernt wird.

Erfindungswesentlich ist es, wie schon erwähnt, daß bereits vom Beginn der Kondensationsreaktion an der Formaldehyd in relativ geringen Konzentrationen, bezogen auf Cokatalysator bzw. gesamtes

Reaktionsgemisch, vorliegt; andererseits soll jedoch die oben angegebene Mindestkonzentration an Formaldehyd niemals unterschritten werden, weil sonst Verbräunungsreaktionen unter Bildung störender Nebenprodukte auftreten. Auch die beanspruchte pH-Führung ist kritisch: Während der Start der Reaktion vorzugsweise bei pH-Werten zwischen 9 und 10 erfolgt (pH-Bereiche oberhalb von 12 sind zu vermeiden, da dann die geringe Menge des beim Start eingesetzten Formaldehyds zu rasch verbraucht und daher die Kondensationsreaktion schwer kontrollierbar wird), führt man die Kondensationsreaktion anschließend durch geeignete Dosierung des Calciumhydroxids in einem pH-Bereich zwischen 7,5 und 9,5, vorzugsweise zwischen 8 und 9, weiter, wobei man vorzugsweise das Reaktionsgemisch ständig bei Siedetemperatur hält. Bei pH-Werten oberhalb von 9,5 ist die Reaktion schwer kontrollierbar; bei pH-Werten unterhalb von 7,5 wird nicht nur die Reaktionszeit stark verläogert, es tritt überraschenderweise auch ein höherer Verbrauch an Calciumhydroxid ein.

Nachdem die gewünschte Menge an wäßriger Formalinlösung bzw. Paraformaldehyd-Dispersion zugesetzt ist, wird die Kondensationsreaktion in an sich bekannter Weise durch Kühlen bzw. Säurezugabe (vorzugsweise mittels Schwefelsäure oder Oxalsäure, weil hierdurch gleichzeitig die Calciumionen ausgefällt werden) abgebrochen. Gegebenenfalls kann jedoch die Kondensationsreaktion auch bei pH-Werten unterhalb von 7 bis zum vollständigen Verbrauch des Formaldehyds weitergeführt werden. Es ist auch möglich, die am Ende der erfindungsgemäßen Reaktion noch vorhandene Restmenge von mindestens 0,5 Gew.-% Formaldehyd, bezogen auf Reaktionsgemisch, durch Reaktion mit methylolierbaren Verbindungen wie z.B. Aminoplastmonomeren, Phenoplastmonomeren, Dialkylphosphiten oder aldolisierbaren Carbonylverbindungen zu entfernen, wie es unten näher erläutert wird. Für viele Anwendungszwecke ist es erforderlich, die so erhaltene Rohformose anschließend durch Überleiten über Anionen- und Kationenaustauscher vollständig zu entsalzen und auf den gewünschten Wassergehalt einzuengen.

Es ist überraschend, daß erfindungsgemäß in bis zu 95%iger Ausbeute und mit hoher Reproduzierbarkeit der durchschnittlichen OH-Funktionalität hochkonzentrierte wäßrige Lösungen von Polyolen, Hydroxyaldehyden und Hydroxyketonen erhalten werden, die völlig farblos sind und daher keiner weiteren Reinigung und Entfärbung bedürfen, während, wie schon erwähnt, bei den Verfahren des Standes der Technik aufgrund von Zersetzungsreaktionen stark gefärbte, störende Nebenprodukte gebildet werden, deren Entfernung nicht oder nur mühsam mit großem zusätzlichem Aufwand gelingt. Abgesehen davon lassen sich diese stark gefärbten Lösungen gemäß Stand der Technik nicht oder nur mühsam und mit geringen Ausbeuten zu mehrwertigen Alkoholen hydrieren, während die katalytische Hydrierung der erfindungsgemäßen farblosen Reaktionsmischungen, nach Entfernung des Katalysators durch einfache Fällungsreaktionen, unter milden Bedingungen gelingt, wie sie allgemein für die katalytische Hydrierung von Zuckern angewandt werden.

Beim erfindungsgemäßen Verfahren wird zunächst in einem Primärschritt aus zwei Molekülen Formaldehyd Glykolaldehyd gebildet. Durch weitere Anlagerung von Formaldehyd entsteht daraus nach folgendem Schema Glycerinaldehyd:

(I) 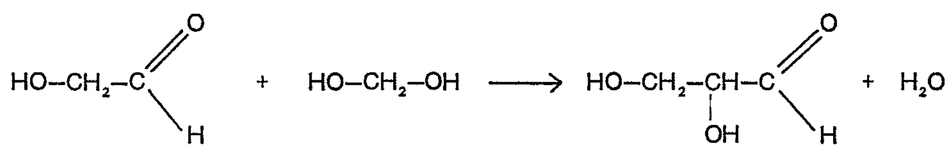

In einer Vielzahl von Folgereaktionen, von denen nur einige wenige beispielhaft genannt sind, entstehen daraus die erfindungsgemäß zugänglichen Gemische von Hydroxyaldehyden und -ketonen:

(II) 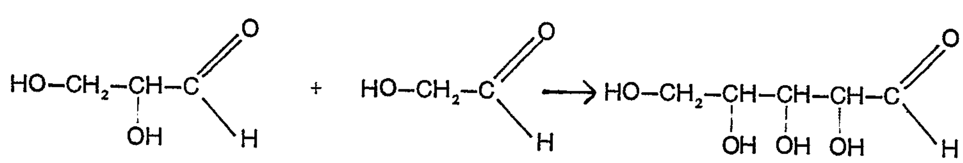

(III) 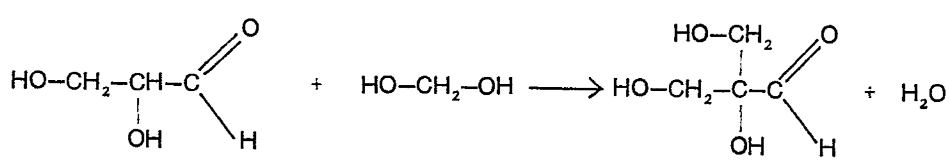

(IV) 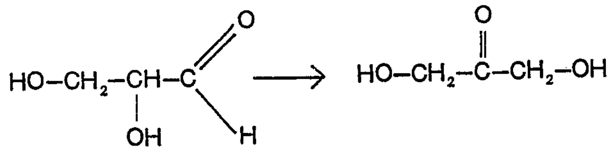

(V) 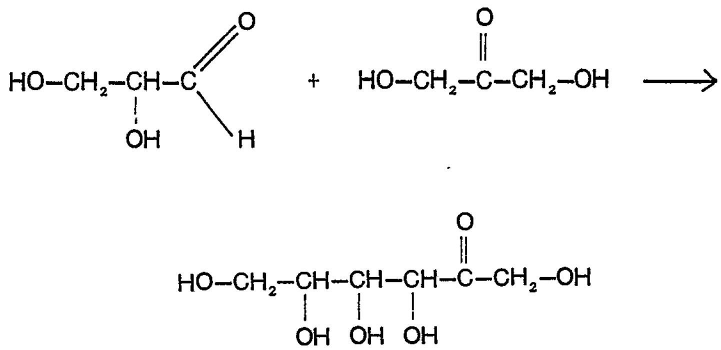

(VI) 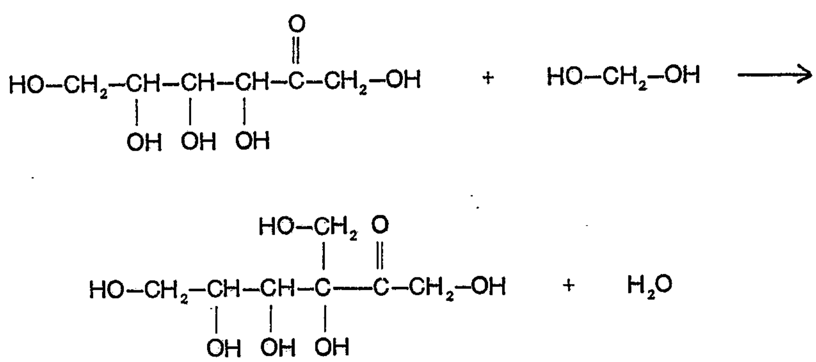

Wie die gaschromatographische Analyse verschiedener erfindungsgemaß hergestellter Produktgemische zeigt, kann mit Hilfe des erfindungsgemäßen Verfahrens einerseits die Produktverteilung variiert werden, wenn man die Reaktion bei verschieden hohen Restformaldehydgehalten abbricht, andererseits ist. die Produktverteilung sowohl im Bereich der Verbindungen mit 2 bis 4 Kohlenstoffatomen, als auch im Bereich mit 5 und mehr Kohlenstoffatomen vollkommen reproduzierbar einzustellen. Dies war aufgrund der Veilzahl der oben nur teilweise genannten Reaktionen, die gleichzeitig und nebeneinander beim erfindungsgemäßen Verfahren ablaufen können, nicht zu erwarten.

Die Kondensation des Formaldehyds erfolgt beim erfindungsgemäßen Verfahren vorzugsweise aus wäßrigen Formaldehydlösungen handelsüblicher Konzentration (30—50 Gew.-% Formaldehyd), die durch Methanol oder andere bekannte Stabilisierungsmittel stabilisiert sind. Es ist jedoch auch möglich, nicht stabilisierte Formaldehydlösungen, die Anteile von festem, polymerisiertem Formaldehyd enthalten, und/oder Paraformaldehyddispersionen zu verwenden, da im Laufe des erfindungsgemäßen Verfahrens diese Feststoffe durch Depolymerisation aufgelöst und ebenfalls zu Hydroxyaldehyden und Hydroxyketonen kondensiert werden. Die Kondensation aus noch höher konzentrierten Formaldehydlösungen, die beispielsweise durch Depolymerisation von Paraformaldehyd oder durch Einengen von Formaldehydlösungen niedriger Konzentration im Vakuum hergestellt werden können, ist ebenfalls möglich. So können beispielsweise Hydroxyaldehyde und Hydroxyketone in sehr guten Ausbeuten durch Kondensation einer 65%igen Formaldehydlösung, die durch Einengen einer 37%igen Formaldehydlösung im Vakuum erhalten wurde, gewonnen werden. Selbstverständlich kann das erfindungsgemäße Verfahren auch auf weniger konzentrierte Formaldehydlösungen angewendet werden, doch ist der Einsatz dieser niedrigkonzentrierten Formaldehydlösungen wegen der zusätzlich erforderlichen Energiekosten für die Verdampfung des Lösungsmittels aus wirtschaftlicher Sicht weniger bevorzugt.

Zum Anfahren der Kondensationsreaktion des Formaldehydhydrats werden erfindungsgemäß wäßrige Cokatalysator-Lösungen verwendet, welche 0,5 bis 10 Gew.-% vorzugsweise 1,2 bis 6 Gew.-%, an Formaldehyd enthalten. Die Konzentration des Cokatalysators in diesem Startergemisch beträgt im allgemeinen 2 bis 90 Gew.-%, vorzugsweise 10 bis 80 Gew.-%, besonders bevorzugt 20 bis 60 Gew.-%. Als Cokatalysator kommen erfindungs-gemäß alle an sich bekannten Endiolgruppen-haltigen Verbindungen bzw. entsprechend der Gleichung

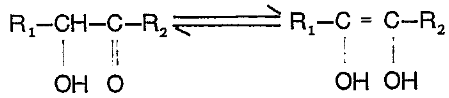

in welcher
$R_1$ und $R_2$ für Wasserstoff, Alkyl, Hydroxyalkyl- oder Aryl-gruppen stehen,
zur Endiolbildung befähigten Verbindungen in Betracht. Beispiele hierfür sind insbesondere Glucose, Fructose, Ascorbinsäure, Benzoin, Glykoaldehyd, Glycerinaldehyd, Erythrose und Invertzucker. Erfindungsgemäß bevorzugte Cokatalysatoren sind jedoch Formosen, insbesondere solche, die durch folgende Molverhältnisse charakterisiert sind:
Verbindungen mit 3 C-Atomen/Verbindungen mit 4 C-Atomen: 0,5—2,0
Verbindungen mit 4 C-Atomen/Verbindungen mit 5 C-Atomen: 0,2—2,0
Verbindungen mit 5 C-Atomen/Verbindungen mit 6 C-Atomen: 0,5—5,0
wobei der Anteil der Komponenten mit 3 bis 6 C-Atomen mindestens 75 Gew.-%, vorzugsweise mehr als 85 Gew.-%, bezogen auf gesamten Cokatalysator, beträgt.

Da alle diese erfindungsgemäß in Frage kommenden Cokatalysatoren zahlreiche freie Hydroxylgruppen enthalten, liegt der beim Start der erfindungsgemäßen Reaktion vorhandene Formaldehyd weitgehend in Form von Halbacetalen mit diesen Hydroxylverbindungen vor.

Das Startergemisch kann neben zur Endiolbilding befähigten Verbindungen und Formaldehyd auch ein- oder mehrwertige Alkohole mit einem Molekulargewicht bis ca. 400 bzw. auch höhermolekulare Polyhydroxylverbindungen enthalten. Als niedermolkulare Alkohole kommen in diesem Zusammenhang beispielsweise Methanol, Äthanol, Isopropanol, n-Butanol, t-Butanol, Neopentylalkohol, Äthylenglykol-Monomethyläther, Äthylenglykol-monoäthyläther, Äthylenglykol, 1,2- und 1,3-Propylenglykol, 1,4- und 2,3-Butylenglykol, 1,6- und 2,3-Hexandiol, 2-Methyl-1,2-propandiol, 1,2,4-Butantriol, 1,2,6-Hexantriol, Glycerin, Erythrit, Chinit, Mannit, Sorbit und Methylglykosid bzw. Anlagerungsprodukte von Äthylenoxid und/oder Propylenoxid an diese Alkohole in Betracht. Bevorzugt sind mehrwertige Alkohole mit mindestens 2 benachbarten Hydroxylgruppen. Geeignete höhermolekulare Polyhydroxylverbindungen sind z.B. solche, die als Ausgangskomponenten für die Herstellung von Polyurethankunststoffen einsetzbar sind. Selbstverständlich können alle diese Polyhydroxylverbindungen auch erst während des erfindungsgemäßen Verfahrens, d.h. gleichzeitig mit dem Formaldehyd und dem Calciumhydroxid, dem Reaktionsgemisch zugesetzt werden.

Gegebenenfalls kann man die erfindungsgemäße Reaktion auch in Abwesenheit von zur Endiolbildung befähigten Verbindungen starten, wenn im Startergemisch Polyole enthalten sind, welche mindestens 2 benachbarte Hydroxylgruppen enthalten. Diese Arbeitsweise ist analog zu jener von DOS 2 714 104.

Je nach der während des erfindungsgemäßen Verfahren eingehaltenen Formaldehydkonzentration im Reaktionsgemisch erhält man Produkte mit unterschiedlicher Funktionalität und mittlerem Molekulargewicht. So werden, wenn man die Formaldehydkonzentration relativ niedrig hält und die Kondensation bis zu einem Restformaldehydgehalt von 0,5 Gew.-% oder nur wenig darüber führt, hauptsächlich Produkte mit 5, 6 oder 7 Kohlenstoffatomen erhalten. Darüberhinaus treten Methylolierungsreaktionen an den zu den Carbonylgruppen der Formose $\alpha$-ständigen Kohlenstoffatomen ein, was zur Bildung verzweigter Zucker führt. Hält man jedoch während des erfindungsgemäßen Verfahrens relativ hohe Formaldehydkonzentrationen (in der Nähe von 10 Gew.-%, bezogen auf gesamtes Reaktionsgemisch) ein, und bricht die Reaktion bei hohen Restformaldehydgehalten ab, so enthält das entstehende Produktgemisch nur wenig Verbindungen mit 6 oder mehr Kohlenstoffatomen. Dagegen ist der Anteil an Verbindungen mit 2 bis 4 Kohlenstoffatomen merklich erhöht. Auf diese Weise lassen sich erfindungsgemäß die verschiedensten Produktverteilungen dadurch erhalten, daß man die Selbstkondensation des Formaldehyds bei verschiedenen Formaldehydkonzentrationen ablaufen läßt bzw. bis zu verschiedenen Restformaldehydgehalten führt. Es läßt sich so jede gewünschte Produktverteilung; die für ein bestimmtes Anwendungsgebiet nötig wird, herstellen.

Gemische mit überwiegenden Anteilen an höhermolekularen Produkten werden auch dadurch erhalten, daß man hydroxyaldehyd- und Hydroxyketongemische, die überwiegend niedermolekulare Anteile enthalten, nachträglich mit überschüssigem Formaldehyd und in Gegenwart einer anorganischen oder organischen Base bei einem pH-Wert von 9 bis 13, vorzugsweise von 10 bis 11, ca. 10 Minuten bis 12 Stunden bei 10—100°C, bevorzugt bei 30—60°C, nachbehandelt. Auf diese Weise werden nicht nur die niedermolekularen Verbindungen durch eine alkalisch katalysierte Aldolreaktion in höhermolekularen Verbindungen übergeführt, sondern auch durch zusätzliche Methylolierung am der Carbonylgruppe benachbarten Kohlenstoffatom in erhöhtem Maße verzweigte Hydroxyaldehyde und Hydroxyketone gebildet. Diese verzweigten Hydroxyketone und Hydroxyaldehyde haven gegenüber den geradkettigen wesentlich mehr primäre Hydroxylgruppen. Die Reaktivität dieser Gemische gegenüber hydroxylgruppenreaktiven Reaktionspartnern ist dadurch deutlich erhöht, was für manche Zwecke von Vorteil ist. So werden beispielsweise bei der Umsetzung der erfindungsgemäß hergestellten Verbindungen mit organischen Isocyanaten infolge der Anwesenheit primärer OH-Gruppen wesentlich schneller Urethane gebildet, als dies mit normalen, geradkettigen, sekundäre OH-Gruppen enthaltenden mehrwertigen Alkoholen der Fall ist.

Für diese nachträgliche gezielte $\alpha$-Methylolierung der erfindungsgemäß erhaltenen Formosen sind insbesondere tertiäre Amine wie z.B. Triäthylamin, Tripropylamin oder Dimethylbenzylamin geeignet.

Aus den im erfindungsgemäßen Verfahren entstehenden Hydroxyaldehyden und Hydroxyketonen können gegebenenfalls nach an sich bekannten Verfahren durch Reduktion in einfacher Weise mehrwertige Alkohole gewonnen werden. So gelingt z.B. die Reduktion direkt aus der erhaltenen wäßrigen Lösung schon bei Raumtemperatur mit Natriumborhydrid; sie kann aber z.B. auch auf elektrolytischem Weg erfolgen. Auch die Katalytische Hydrierung mit Wasserstoff ist möglich. Hierfür können prinzipiell alle Verfahren, die bei der Reduktion von Zuckern zu Zuckeralkoholen zum Stand der Technik gehören, angewandt werden. Besonders günstig ist die Hydrierung mit Raney-Nickel in Mengen von 5-20 Gew.-%, bezogen auf zu reduzierendes Hydroxyaldehyd- und Hydroxyketongemisch, bei Wasserstoffdrucken von 50—200 kgg/cm$^2$ und Temperaturen von 20—200°C, jedoch können mit ähnlich gutem Erfolg auch Katalysatoren, die Nickel, Kobalt, Kupfer, Platin, Rhodium oder Palladium auf inerten Trägern enthalten, verwendet werden. Diese reduzierten Formosen werden im folgenden "Forite" genannt.

Es ist jedoch auch möglich, die in den erfindungsgemäß erhaltenen Formosen vorhandenen Hydroxyaldehyde und -ketone nachträglich mit Formaldehyd zu reduzieren. Dazu wird die Reaktionslösung mit überschüssigem Formaldehyd und einer anorganischen Base versetzt und 30 Minuten bis 12 Stunden lang bei 10 bis 100°C, vorzugsweise 30 bis 60°C, unter Einhaltung eines pH-Wertes von 9 bis 13, vorzugsweise von 10 bis 11, gerührt. Es ist dabei möglich, nicht nur die Carbonylfunktion zu reduzieren, sondern gleichzeitig, wie oben erläutert, höhermolekulare und verzweigte Produkte zu synthetisieren. Bevorzugte anorganische Basen, die die gekreuzte Cannizzaro-Reaktion beschleunigen, sind Natriumhydroxid, Kaliumhydroxid, Calcium- und Bariumhydroxid sowie "Kronenäther"-Komplexe von Alkaliatomen.

Die Reduktionsreaktion kann durch Co-Katalysatoren noch weiter beschleunigt werden. Bevorzugt sind in diesem Zusammenhang Oxalate von Übergangsmetallen, insbesondere Nickel-, Kobalt-, Eisen-, Cadmium-, Zink-, Chrom- und Manganoxalat sowie Übergangsmetalle in elementarer Form, z.B. Nickel, Kobalt, Eisen, Kupfer, Cadmium, Zink, Chrom und Mangan. Ganz besonders bevorzugt sind aktiviertes Nickel, das in Form von sogenanntem Raney-Nickel eingesetzt wird, und elementares Zink in Pulverform.

Als weitere Co-Katalysatoren für die Reduktion mittels Formaldehyd kommen Amide organischer Säuren, wie Formamid, Dimethylformamid und Acetamid sowie Tetraalkylammoniumsalze, insbesondere Tetramethylammoniumchlorid und Tetraäthylammoniumchlorid, in Frage.

Wie schon mehrfach erwähnt, kann der am Ende des erfindungsgemäßen Verfahrens in der Formose noch vorhandene Restformaldehyd nicht nur durch Weiterkondensation im neutralen bzw. schwach basischen pH-Bereich sondern auch durch Zusatz von methylolierbaren Verbindungen entfernt werden. Solche Verbindungen sind beispielsweise Aldehyde und Ketone, welche in $\alpha$-Stellung zur Carbonylgruppe ein Wasserstoffatom aufweisen, wie Acetaldehyd, Butyraldehyd, Isobutyraldehyd, Methyläthylketon, Aceton, Cyclopentanon, Cyclohexanon, Mesityloxid, Isophoron, Acetophenon und Acetessigester. Bevorzugt sind in diesem Zusammenhang Butyraldehyd, Isobutyraldehyd, Aceton und Cyclohexanon.

Zum Abfangen des restlichen Formaldehyds kommen jedoch erfindungsgemäß auch zur Aminoplastbildung befähigte Verbindungen in Betracht, welche mit Formaldehyd unter Bildung der entsprechenden N-Methylolderivate reagieren. Geeignete Aninoplastmonomere werden beispielsweise in der DOS 2 324 134 beschrieben. Bevorzugt sind in diesem Zusammenhang Harnstoff, Thioharnstoff, $\varepsilon$-Caprolactam, Bisurethane, Oxamid, Pyrrolidon, Dicyandiamid, Melamin, Phenole, Naphthole, Bisphenol A, Phenol- und Naphtholsulfonate.

Der Restformaldehyd kann schließlich auch durch Zusatz von Dialkylphosphiten, insbesondere Dimethylphosphit und Diäthylphosphit, entfernt werden, wobei sich die entsprechenden Hydroxymethylphosphonsäureester bilden.

Der Zusatz der genannten methylolierbaren Verbindungen hat den Vorteil, daß durch sie die Viskosität der erfindungsgemäß erhaltenen Formosen erheblich reduziert wird, Selbstverständlich ist es, um diesen Effekt zu erzielen, erfindungsgemäß auch möglich, die erwähnten methylolierbaren Verbindungen (bzw. auch schon deren Methylolierungsprodukte) bereits beim Start der Kondensationsreaktion oder zu einem beliebigen Zeitpunkt während des erfindungsgemäßen Verfahrens zuzusetzen.

Die erfindungsgemäß zugänglichen Gemische von hydroxyaldehyden und Hydroxyketonen bzw. daraus durch gekreuzte Cannizzaro-Reaktion oder Hydrierung entstandenen mehr- wertigen Alkohole sind wertvolle Ausgangsmaterialien für eine Vielzahl anwendungstechnisch interessanter Produkte.

Beispielsweise sind die durch Reduktion erhaltenen Polyhydroxylverbindungen sehr gut als Kettenverlängerungsmittel bzw. Vernetzer bei der Herstellung von Polyurethankunststoffen aus Polyisocyanaten, niedermolekularen Polyhydroxylverbindungen sowie gegebenenfalls höhermolekularen Polyhydroxylverbindungen, weiteren Kettenverlängerungsmitteln, Treibmitteln, Katalysatoren und weiteren an sich bekannten Zusatzstoffen geeignet. Als Polyisocyanate kommen in diesem Zusammenhang beispielsweise die aliphatischen, cycloaliphatischen, araliphatischen, aromatischen und heterocyclischen Polyisocyanate in Frage, wie sie z.B. von W. Siefken in Justus Liebigs Annalen der Chemie, 562, Seiten 75 bis 136, beschrieben werden, beispielsweise Äthylen-diisocyanat, 1,4-Tetramethylen-

diisocyanat, 1,6-Hexamethylendiisocyanat, 1,12 Dodecandiisocyanat, Cyclobutan-1,3-diisocyanat, Cyclohexan-1,3- und -1,4-Diisocyanat sowie beliebige Gemische dieser Isomeren, 1-Isocyanato-3,3,5-trimethyl-5-isocyanatomethyl-cyclohexan (DAS 1 202 785, amerikanische patentschrift 3 401 190), 2,4- und 2,6-Hexahydrotolylendiisocyanat sowie beliebige Gemische dieser Isomeren, Hexahydro-1,3- und/oder -1,4-phenylen-diisocyanat, Perhydro-2,4'- und/oder -4,4'-diphenylmethan-diisocyanat, 1,3- und 1,4-Phenylendiisocyanat, 2,4- und 2,6-Toluylendiisocyanat sowie beliebige Gemische dieser Isomeren, Diphenylmethan-2,4'- und/oder -4,4'-diisocyanat, Naphthylen-1,5-diisocyanat, Triphenylmethan-4,4',4''-triisocyanat, Polyphenyl-polymethylen-polyisocyanate, wie sie durch Anilin-Formaldehyd-Kondensation und anschließende Phosgenierung erhalten und z.B. in den britischen Patentschriften 874 430 und 848 671 beschrieben werden, m- und p-Isocyanatophenylsulfonyl-isocyanate gemäß der amerikanischen Patentschrift 3 454 606, perchlorierte Arylpolyisocyanate, wie sie z.B. in der deutschen Auslegeschrift 1 157 601 (amerikanische Patentschrift 3 277 138) beschrieben werden, Carbodiimidgruppen aufweisende Polyisocyanate, wie sie in der deutschen Patentschrift 1 092 007 (amerikanische Patentschrift 3 152 162) beschrieben werden, Diisocyanate, wie sie in der amerikanischen Patentschrift 3 492 330 beschrieben werden, Allophanatgruppen aufweisende Polyisocyanate, wie sie z.B. in der britischen Patentschrift 994 890, der belgischen Patentschrift 761 626 und der veröffentlichten holländischen Patentanmeldung 7 102 524 beschrieben werden, Isocyanuratgruppen aufweisende Polyisocyanate, wie sie z.B. in der amerikanischen Patentschrift 3 001 973, in den deutschen Patentschriften 1 022 789, 1 222 067 und 1 027 394 sowie in den deutschen Offenlegungsschriften 1 929 034 und 2 004 048 beschrieben werden, Urethangruppen aufweisende Polyisocyanate, wie sie z.B. in der belgischen Patentschrift 752 261 oder in der amerikanischen Patentschrift 3 394 164 beschrieben werden, acylierte Harnstoffgruppen aufweisende Polyisocyanate gemäß der deutschen Patentschrift 1 230 778, Biuretgruppen aufweisende Polyisocyanate, wie sie z.B. in der deutschen Patentschrift 1 101 394 (amerikanische Patentschriften 3 124 605 und 3 201 372) sowie in der britischen Patentschrift 889 050 beschrieben werden, durch Telomerisationsreaktionen hergestellte Polyisocyanate, wie sie z.B. in der amerikanischen Patentsschrift 3 654 106 beschrieben werden, Estergruppen aufweisende Polyisocyanate, wie sie zum Beispiel in den britischen Patentschriften 965 474 und 1 072 956, in der amerikanischen Patentschrift 3 567 763 und in der deutschen Patentschrift 1 231 688 genannt werden, Umsetzungsprodukte der obengenannten Isocyanate mit Acetalen gemäß der deutschen Patentschrift 1 072 385 und polymere Fettsäurereste enthaltende Polyisocyanate gemäß der amerikanischen Patentschrift 3 455 883.

Es ist möglich, die der technischen Isocyanatherstellung anfallenden, Isocyanatgruppen aufweisenden Destillationsrück-stände, gegebenenfalls gelöst in einem oder mehreren der vorgenannten Polyisocyanate, einzusetzen. Ferner ist es möglich, beliebige Mischungen der vorgenannten Polyisocyanate zu verwenden.

Besonders bevorzugt werden in der Regel die technisch leicht zugänglichen Polyisocyanate, z.B. das 2,4- und 2,6-Toluylendiisocyanat sowie beliebige Gemische dieser Isomeren ("TDI"), Polyphenylpolymethylen-polyisocyanate, wie sie durch Anilin-Formaldehyd-Kondensation und anchließende Phosgenierung hergestellt werden ("rohes MDI") und Carbodiimidgruppen, Urethangruppen, Allophenatgruppen, Isocyanuratgruppen, Harnstoffgruppen oder Biuretgruppen aufweisenden Polyisocyanate ("modifizierte Polyisocyanate").

Geeignete höhermolekulare Polyhydroxylverbindungen, speziell solche vom Molekulargewicht 800 bis 10 000, vorzugsweise 1000 bis 6000, sind z.B. mindestens zwei, in der Regel 2 bis 8, vorzugsweise aber 2 bis 4, Hydroxylgruppen aufweisende Polyester, Polyäther, Polythioäther, Polyacetale, Polycarbonate und Polyesteramide, wie sie für die Herstellung von homogenen und von zellförmigen Polyurethanen an sich bekannt sind.

Die in Frage kommenden Hydroxylgruppen aufweisenden Polyester sind z.B. Umsetzungsprodukte von mehrwertigen, vorzugsweise zweiwertigen und gegebenenfalls zusätzlich dreiwertigen Alkoholen mit mehrwertigen, vorzugsweise zweiwertigen, Carbonsäuren. Anstelle der freien Polycarbonsäuren können auch die entsprechenden Polycarbonsäureanhydride oder entsprechende Polycarbonsäureester von niedrigen Alkoholen oder deren Gemische zur Herstellung der Polyester verwendet werden. Die Polycarbonsäuren können aliphatischer, cycloaliphatischer, aromatischer und/oder heterocyclischer Natur sein und gegebenenfalls, z.B. durch Halogenatome, substituiert und/oder ungesättigt sein.

Als Beispiele hierfür seien genannt: Bernsteinsäure, Adipinsäure, Korksäure, Azelainsäure Sebacinsäure, Phthalsäure, Isophthalsäure, Trimellitsäure, Phthalsäureanhydrid, Tetrahydrophthalsäureanhydrid, Hexahydrophthalsäureanhydrid, Tetrachlorphthalsäureanhydrid, Endomethylentetrahydrophthalsäureanhydrid, Glutarsäureanhydrid, Maleinsäure, Maleinsäureanhydrid, Fumarsäure, dimere und trimere Fettsäuren wie Ölsäure, gegebenenfalls in Mischung mit monomeren Fettsäuren, Terephthalsäuredimethylester und Terephthalsäure-bis-glykolester. Als mehrwertige Alkohole kommen z.B. Äthylenglykol, Propylenglykol-(1,2) und -(1,3), Butylenglykol-(1,4) und -(2,3), Hexandiol-(1,6), Octandiol-(1,8), Neopentylglykol, Cyclohexandimethanol (1,4-Bis-hydroxymethylcyclohexan), 2-Methyl-1,3-propandiol, Glycerin, Trimethylolpropan, Hexantriol-(1,2,6), Butantriol-(1,2,4) Trimethyloläthan, Pentaerythrit, Chinit, Mannit und Sorbit, Methylglykosid, ferner Diäthylenglykol, Triäthylenglykol, Tetraäthylenglykol, Polyäthylenglykole, Dipropylenglykol, Polypropylenglykole, Dibutylenglykol

und Polybutylenglykole in Frage. Die Polyester können anteilig endständige Carboxylgruppen aufweisen, Auch Polyester aus Lactonen, z.B. ε-Caprolacton oder Hydroxycarbonsäuren, z.B. ω-Hydroxycapronsäure, sind einsetzbar.

Auch die in Frage kommenden, mindestens zwei, in der Regel zwei bis acht, vorzugsweise zwei bis drei, Hydroxylgruppen aufweisenden Polyäther sind solche der an sich bekannten Art und werden z.B. durch Polymerisation von Epoxiden wie Äthylenoxid, Propylenoxid, Butylenoxid, Tetrahydrofuran, Styroloxid oder Epichlorhydrin mit sich selbst, z.B. in Gegenwart von $BF_3$, oder durch Anlagerung dieser Epoxide, gegebenenfalls im Gemisch oder nacheinander, an Startkomponenten mit reaktionsfähigen Wasserstoffatomen wie Wasser, Alkohole, Ammoniak oder Amine, z.B. Äthylenglykol, Propylenglykol-(1,3) oder -(1,2), Trimethylolpropan 4,4'-Dihydroxy-diphenylpropan, Anilin, Äthanolamin oder Äthylendiamin hergestellt. Auch Sucrosepolyäther, wie sie z.B. in den deutschen Auslegeschriften 1 176 358 und 1 064 938 beschrieben werden, kommen hierfür in Frage. Vielfach sind solche Polyäther bevorzugt, die überweigend (bis zu 90 Gew.-%, bezogen auf alle vorhandenen OH-Gruppen im Poly-äther) primäre OH-Gruppen aufweisen. Auch durch Vinylpolymerisate modifizierte Polyäther, wie sie z.B. durch Polymerisation von Styrol und Acrylnitril in Gegenwart von Polyäthern entstehen (amerikanische Patentschriften 3 383 351, 3 304 273, 3 523 093, 3 110 695, deutsche Patentschrift 1 152 536), sind geeignet, ebenso OH-Gruppen aufweisende Polybutadiene.

Unter den Polythioäthern seien insebesondere die Kondensationsprodukte von Thiodiglykol mit sich selbst und/oder mit anderen Glykolen, Dicarbonsäuren, Formaldehyd, Aminocarbonsäuren oder Aminoalkoholen angeführt. Je nach den Co-Komponenten handelt es sich bei den Produkten um Polythiomischäther, Polythioätherester oder Polythioätheresteramide.

Als Polyacetale kommen z.B. die aus Glykolen, wie Diäthylenglykol, Triäthylenglykol, Triäthylenglykol, 4,4'-Dioxäthoxydiphenyldimethylmethan, Hexandiol und Formaldehyd herstellbaren Verbindungen in Frage. Auch durch Polymerisation cyclischer Acetale lassen sich hierfür geeignete Polyacetale herstellen.

Als Hydroxylgruppen aufweisende Polycarbonate kommen solche der an sich bekannten Art in Betracht, die z.B.durch Umsetzung von Dilen wie Propandiol-(1,3), Butandiol-(1,4) und/oder Hexandiol-(1,6), Diäthylenglykol, Triäthylenglykol oder Tetraäthylenglykol mit Diarylcarbonaten, z.B. Diphenylcarbonat, oder Phosgen hergestellt werden können.

Zu den Polyesteramiden und Polyamiden zählen z.B. die aus mehrwertigen gesättigten und ungesättigten Carbonsäuren bzw. deren Anhydriden und mehrwertigen gesättigten und ungesättigten Aminoalkoholen, Diaminen, Polyaminen und ihren Mischungen gewonnenen, vorwiegend linearen Kondensate.

Auch bereits Urethan- oder Harnstoffgruppen enthaltende Polyhydroxylverbindungen sowie gegebenenfalls modifizierte natürliche Polyole, wie Rizinusöl, Kohlenhydrate oder Stärke, sind verwendbar. Auch Anlagerungsprodukte von Alkylenoxiden an Phenol-Formaldehyd-Harze oder auch an Harnstoff-Formaldehydharze sind hierfür einsetzbar.

Vertreter dieser gegebenenfalls mit zuverwendenden Verbindungen sind z.B. in High Polymers, Vol. XVI, "Polyurethanes, Chemistry and Technology", verfaßt von Saunders-Frisch, Interscience Publishers, New York, London, Band I, 1962, Seiten 32—42 und Seiten 44—54 und Band II, 1964, Seiten 5—6 und 198—199, sowie im Kunstoff-Handbuch, Band VII, Vieweg-Höchtlen, Carl-Hanser-Verlag, München, 1966, z.B. auf den Seiten 45—71, beschrieben.

Selbstverständlich können Mischungen der obengenannten Verbindungen mit mindestens zwei gegenüber Isocyanaten reaktionsfähigen Wasserstoffatomen mit einem Molekulargewicht von 800—10 000, z.B. Mischungen von Polyäthern und Polyestern, eingesetzt werden.

Als gegebenenfalls einzusetzende Ausgangskomponenten kommen auch Verbindungen mit mindestens zwei gegenüber Isocyanaten reaktionsfähigen Wasserstoffatomen von einem Molekulargewicht 32—400 in Frage. Auch in diesem Fall versteht man hierunter Hydroxylgruppen und/oder Aminogruppen und/oder Thiolgruppen und/oder Carboxylgruppen aufweisende Verbindungen, vorzugsweise Hydroxylgruppen und/oder Aminogruppen aufweisende Verbindungen, die als Kettenverlängerungsmittel oder Vernetzungsmittel dienen. Diese Verbindungen weisen in der Regel 2 bis 8 gegenüber Isocyanaten reaktionsfähige Wasserstoffatome auf, vorzugsweise 2 oder 3 reaktionsfähige Wasserstoffatome.

Als Beispiele für derartige Verbindungen seien genannt: Äthylenglykol, Propylenglykol-(1,2) und -(1,3), Butylenglykol-(1,4) und -(2,3), Pentandiol-(1,5), Hexandiol-(1,6), Octandiol-(1,8), Neopentylglykol, 1,4-Bis-hydroxymethyl-cyclohexan, 2-Methyl-1,3-propandiol, Glyzerin, Trimethylolpropan, Hexantriol-(1,2,6), Trimethyloläthan, Pentaerythrit, Chinit, Mannit und Sorbit, Diäthylenglykol, Triäthylenglykol, Tetraäthylenglykol, Polyäthylenglykole mit einem Molekulargewicht bis 400, Dipropylenglykol, Polypropylenglykole mit einem Molekulargewicht bis 400, Dibutylenglykol, Polybutylenglykole mit einem Molekulargewicht bis 400, 4,4'-Dihydroxydiphenylpropan, Di-hydroxymethyl-hydrochinon, Äthanolamin, Diäthanolamin, Triäthanolamin, 3-Aminopropanol, Äthylendiamin, 1,3-Diaminopropan, 1-Mercapto-3-aminopropan, 4-Hydroxy- oder -Amino-phthalsäure, Bernsteinsäure, Adipinsäure, Hydrazin, N,N'-Dimethylhydrazin, 4,4'-Diaminodiphenylmethan, Toluylendiamin, Methylen-bis-chloranilin, Methylen-bis-anthranilsäureester Diaminobenzoesäureester und die isomeren Chlorphenylendiamine.

Auch in diesem Fall können Mischungen von verschiedenen Verbindungen mit mindestens zwei gegenüber Isocyanaten reaktionsfähigen Wasserstoffatomen mit einem Molekulargewicht von 32—400 verwendet werden.

Hierfür können jedoch auch Polyhydroxylverbindungen eingesetzt werden, in welchen hochmolekulare Polyaddukte bzw. Polykondensate in feindisperser oder gelöster Form enthalten sind. Derartige modifizierte Polyhydroxylverbindungen werden erhalten, wenn man Polyadditionsreaktionen (z.B. Umsetzungen zwischen Polyisocyanaten und aminofunktionellen Verbindungen) bzw. Polykondensationsreaktionen (z.B. zwischen Formaldehyd und Phenolen und/oder Aminen) direkt in situ in den oben genannten, Hydroxylgruppen aufweisenden Verbindungen ablaufen läßt. Derartige Verfahren sind beispielswiese in den Deutschen Auslegeschriften 1 168 075 und 1 260 142, sowie den Deutschen Offenlegungsschriften 2 324 134, 2 423 984, 2 512 385, 2 513 815, 2 550 796, 2 550 797, 2 550 833 und 2 550 862 beschrieben. Es ist aber auch möglich, gemäß US-Patent 3 869 413 bzw. Deutscher Offenlegungsschrift 2 550 860 eine fertige wäßrige Polymerdispersion mit einer Polyhydroxylverbindung zu vermischen und anschließend aus dem Gemisch das Wasser zu entfernen.

Bei der Verwendung von modifizierten Polyhydroxylverbindungen der oben genannten Art als Ausgangskomponente im Polyisocyanat-Polyadditionsverfahren entstehen in vielen Fällen Polyurethankunststoffe mit wesentlich verbesserten mechanischen Eigenschaften.

Die ausschließliche Umsetzung der erfindungsgemäß zugänglichen Polyhydroxylverbindungen (ohne Mitverwendung anderer gegenüber Isocyanaten reaktiver Komponenten) mit stark elastifizierenden Polyisocyanaten, wie z.B. Polyisocyanaten mit Biuretstruktur (DAS 1 543 178) führt zu harten, lichtechten, kratz- und lösungsmittelfesten Beschichtungen und Lacken.

Gegebenenfalls zellförmige Polyurethankunststoffe können durch Umsetzung von

a) Polyisocyanaten mit
b) Polyhydroxylverbindungen mit einem Molekulargewicht unter 400 sowie gegebenenfalls
c) höhermolekularen Polyhydroxylverbindungen und/oder weiteren Kettenverlängerungsmitteln, gegebenenfalls in Gegenwart von
d) Treibmitteln, Katalysatoren und weiteren an sich bekannten Zusatzstoffen,

hergestellt werden, wobei als Komponente b) die erfindungsgemäß hergestellten Formosen bzw. die daraus durch Reduktion erhaltenen Formite eingesetzt werden.

Durch Propoxylierung oder/und Oxyäthylierung der Formosen bzw. der Formite lassen sich ferner Polyätheralkohole hoher Funktionalität gewinnen, die in hohen OH-Zahl-Bereichen für die Herstellung von harten bzw. halbharten zellförmigen Polyurethankunststoffen und bei niedrigen OH-Zahlen als Ausgangsmaterialien für hochelastische Polyurethanschaumstoffe Verwendung finden.

Durch Umsetzung der erfindungsgemäß hergestellten Gemische aus mehrwertigen Alkoholen mit mehrwertigen Carbonsäuren der oben genannten Art, z.B. Phthalsäure, Isophthalsäure, Terephthalsäure, Tetra- und Hexahydrophthalsäure, Adipinsäure oder Maleinsäure nach den üblichen Verfahren der Polyesterkondensation, wie sie beispielsweise in Houben-Weyl, Methoden der organischen Chemie, Bd. XIV 12, S. 40 beschrieben sind, lassen sich stark verzweigte Polyester synthetisieren, die als Zusätze zu Alkydharzen deren Härte verbessern. Die Hydroxylgruppen enthaltende Polyester, die aus den erfindungsgemäß hergestellten Hydroxylverbindungen synthetisiert werden, sind selbstverständlich ebenfalls als Ausgangskomponente zur Herstellung von Polyurethankunststoffen brauchbar.

Die erfindungsgemäß hergestellten mehrwertigen Alkohole sowie die Hydroxyaldehyde und Hydroxyketone lassen sich auch sehr leicht mit langkettigen, aliphatischen Monocarbonsäuren, wie Capryl-, Caprin-, Laurin-, Myristin-, Palmitin-, Stearin-, Öl-, Linol-, Arachidon-, oder Behensäure, sowie deren Derivaten, wie z.B. den Methyl- oder Äthylestern oder auch den Anhydriden bzw. gemischten Anhydriden zu hydroxylgruppenhaltigen Estern umsetzen. Diese stellen ebenso wie Oxäthylierungsprodukte der Polyole oder auch Umsetzungsprodukte der erfindungsgemäß zugänglichen Polyhydroxylverbindungen mit langkettigen Monoisocyanaten, wie n-Octyl-, n-Decyl-, n-Dodecyl-, Myristyl-, Cetyl- oder Stearylisocyanat zu Carbamidsäureestern (siehe z.B. K. Lindner, Tenside Bd. III, Wissenschaftliche Verlagsgesellschaft Stuttgart, 1964, S. 2336) nichtionogene, oberflächenaktive Verbindungen dar, die als wertvolle Emulgatoren, Netzmittel oder Weichmacher Verwendung finden können.

Die erfindungsgemäßen Verbindungen lassen sich auch als Feuchthaltemittel in Kosmetika und Kunststoffen verwenden. Sie können aber z.B. auch als Gefrierschutzmittel dienen.

Ebenso ist ihr Einsatz als kohlenhydrathaltiges Substrat in Nährböden von Mikroorganismen möglich. Hierzu haben sich besonders diejenigen Verfahrensprodukte bewährt, die hauptsächlich aus 5 und 6 Kohlenstoffatome enthaltenden Hydroxyaldehyden und Hydroxyketonen bestehen.

Die folgenden Beispiele erläutern das erfindungsgemäße Verfahren. Wenn nicht anders vermerkt, sind Zahlenwerte als Gewichtsteile bzw. Gewichtsprozente zu verstehen.

### Beispiel 1

In einem 2 l Mehrhalskolben, der mit einer Destillationsbrücke mit Vorlagekolben, einer pH-Elektrode, einem Rührer und zwei Tropftrichtern versehen ist, werden 300 g einer gemäß Beispiel 1 von DOS 2 639 084 hergestellten, vollentsalzten 25%igen wäßrigen Formoselösung vorgelegt. Die wäßrige

Formose wird auf 95 bis 97°C aufgeheizt. In die siedende Lösung werden dann 50 ml einer 37%igen Formalinlösung aus einem der Tropftrichter zudosiert. Nachdem wieder Siedetemperatur der Mischung erreicht ist, wird die Heizquelle entfernt. Aus dem zweiten Tropftrichter dosiert man dann so viel einer 25%igen wäßrigen $Ca(OH)_2$-Suspension zu, daß sich der pH der Mischung auf 9,8 einstellt. Das zudosierte $Ca(OH)_2$ geht in Lösung, die Reaktionsmischung färbt sich grün bis gelblich und beginnt ohne zusätzliche Heizquelle mäßig zu sieden. Nachdem die Reaktion angesprungen ist, wird in dem Maße $Ca(OH)_2$-Suspension und Formalinlösung gleichzeitig zudosiert, daß die Mischung ständig mäßig siedet, der pH der Reaktionslösung im Bereich zwischen 8 und 9 bleibt und die Formaldehydkonzentration ca. 4—5% beträgt.

Nach Zugabe von 1000 g 37%iger Formalinlösung wird die $Ca(OH)_2$-Zugabe nach 30 Minuten gestoppt und der Formaldehydgehalt der Mischung zu 4,4% bestimmt. Nach 37 Minuten Gesamtreaktionszeit ist der Formaldehydgehalt der Mischung auf, 3,3% abgesunken. Insgesamt wurden 30 g $Ca(OH)_2$ zudosiert.

Durch Zugabe von 199 g 20%iger Schwefelsäure wird das Ca aus der Mischung ausgefällt. Nach Filtrieren und Einengen im Vakuum werden 419 g (89% der Theorie) einer hellfarbigen Formose erhalten, welche 5,4% Wasser enthält und einen Zuckergehalt von 57,1%, berechnet als Glucose, aufweist.

Nach Hydrierung der Formose und Silylierung ergibt sich aus der gaschromatographischen Analyse folgende Komponentenverteilung:

| | $C_2$ | $C_3$ | $C_4$ | $C_5$ | $C_6$ | $C_7$ | $C_8$ |
|---|---|---|---|---|---|---|---|
| % | 0,10 | 1,04 | 5,60 | 13,38 | 39,42 | 33,49 | 6,98 |

Vergleichsbeispiel 1

500 g einer 37%igen Formalinlösung und 150 g einer 30%igen vollentsalzten Formose gemäß Beispiel 1 von DOS 2 639 084 werden gemischt und auf 60°C aufgeheizt. Der pH der Mischung beträgt 3,1. Durch Zugabe von 15 g pulverisiertem $Ca(OH)_2$ wird der pH der Mischung auf 9,8 angehoben. Durch die spontan einsetzende stark exotherme Reaktion erwärmt sich der Ansatz selbst auf 80°C. Hierauf wurde mit einem vorbereiteten Eisbad intensiv gekühlt. Trotz intensiver Kühlung wird die Reaktion immer heftiger, so daß die Reaktionsmischung durch den Rückflußkühler und aus einer Schliföffnung — nachdem ein Stopfen herausgeschleudert wurde — aus dem Reaktionsgefäß herausspritzte.

Vergleichsbeispiel 2

1000 g einer 37%igen Formalinlösung werden mit 300 g einer vollentsalzten Formose gemäß Beispiel 1 von DOS 2 639 084 gemischt und auf 60°C aufgeheizt. Durch Zugabe von 10 g $Ca(OH)_2$ wird der pH der Mischung auf 7,5 bis 8 eingestellt. Es wird dann in dem Maße $Ca(OH)_2$ nachdosiert, daß der pH der Mischung ständig in diesem Bereich bleibt. Nach insgesamt 96,5 Stunden und Zugabe von 105 g $Ca(OH)_2$ ist der Formaldehydgehalt der Mischung auf 0 abgesunken.

Der Ansatz wird abgekühlt und über Ionenaustauscher entsalzt Nach dem Einengen am Rotationsverdampfer erhält man 317 g einer Formose mit einem Wassergehalt von 7,2% und einem Zuckergehalt von 10,5%, berechnet als Glucose.

Nach Hydrierung und Silylierung ergibt sich aus der gaschromatographischen Analyse forlgende Komponentenverteilung:

| | $C_2$ | $C_3$ | $C_4$ | $C_5$ | $C_6$ | $C_7$ | $C_8$ |
|---|---|---|---|---|---|---|---|
| % | 0,084 | 0,762 | 10,15 | 5,17 | 27,49 | 49,46 | 6,87 |

Das Vergleichsbeispiel zeigt die wesentlich längere Reaktionszeit und geringere Zuckerausbeute bei einer vom erfindungsgemäßen Verfahren abweichenden pH-Führung.

Beispiel 2

In einem 2 1 Mehrhalskolben, der mit einer Destillationsbrücke mit Vorlagekolben, einer pH-Elektrode, einem Rührer und zwei Tropftrichtern versehen ist, werden 75 g reine Glucose und 225 g Wasser vorgelegt. Die wäßrige Lösung wird auf 95°C aufgeheizt. In die siedende Lösung werden dann 50 ml einer 37%igen Formalinlösung aus einem der Tropftrichter zudosiert. Nachdem wieder Siedetemperatur der Mischung erreicht ist, wird die Heizquelle entfernt. Aus dem zweiten Tropftricher dosiert man dann so viel einer 25%igen wäßrigen $Ca(OH)_2$-Suspension zu, daß sich der pH der Mischung auf 9,2 einstellt. Das zudosierte $Ca(OH)_2$ geht in Lösung, die Reaktionsmischung färbt sich gelblich und beginnt ohne zusätzliche Heizquelle mäßig zu sieden. Nachdem die Reaktion angesprungen ist, wird in dem Maße $Ca(OH)_2$-Suspension und Formalin gleichzeitig zudosiert, daß die Mischung ständig mäßig siedet, der pH der Reaktionslösung zwischen 8 und 9 bleibt und die Formaldehydkonzentration 3—4% beträgt.

Nach Zugabe von 1000 g Formalinlösung wird die $Ca(OH)_2$-Zugabe gestoppt. Nach 30 Minuten Gesamtreaktionszeit ist der Formaldehydgehalt der Mischung auf 2% abgesunken. Insgesamt wurden 22,5 g $Ca(OH)_2$ zudosiert.

Durch Zugabe von 149 g 20 %iger Schwefelsäure wird das Ca aus der Mischung ausgefällt. Nach Filtrieren und Einengen im Vakuum wird eine hellfarbige Formose erhalten.

Während der Reaktion destillieren insgesamt 24 g wäßriges Methanol über, welches 2,2% Formaldehyd enthält.

## Patentansprüche

1. Verfahren zur Herstellung von niedermolekularen Polyhydroxylverbindungen durch Kondensation von Formaldehydhydrat in Gegenwart von Calciumhydroxid als Katalysator sowie von zur Endiolbildung befähigten Verbindungen als Cokatalysator, dadurch gekennzeichnet, daß man eine Formaldehyd enthaltende Lösung des Cokatalysators in Wasser und gegebenenfalls niedermolekularen ein- oder mehrwertigen Alkoholen und/oder höhermolekularen Polyhydroxylverbindungen bei einer Temperatur von 80 bis 110°C durch Zusatz von Calciumhydroxid auf einen pH-Wert von 9 bis 12 bringt, so daß die Kondensation des Formaldehydhydrats gestartet wird, anschließend eine 20 bis 65 Gew.-% Formaldehyd enthaltende wäßrige Formalinlösung und/oder Paraformaldehyd-Dispersion sowie Calciumhydroxid so zudosiert, daß das Reaktionsgemisch bei einer Temperatur von 80 bis 110°C und in einem pH-Bereich zwischen 7,5 und 9,5 gehalten wird, wobei während des ganzen Verlaufs der Kondensationsreaktion die Formaldehydkonzentration zwischen 0,5 und 10 Gew.-%, bezogen auf gesamtes Reaktionsgemisch, gehalten wird und schließlich gegebenenfalls die Restmenge von 0,5 bis 10 Gew.-% Formadehyd durch weitere Kondensation bei pH-Werten unter 7 oder durch Reaktion mit anderen gegenüber Formaldehydhydrat reaktiven Verbindungen entfernt wird.

2. Verfahren nach Anspruch 1, dadurch gekennzeichnet, daß während der Startphase der Reaktion ein pH-Wert zwischen 9 und 10 und danach zwischen 8 und 9 eingehalten wird.

3. Verfahren nach Anspruch 1 oder 2, dadurch gekennzeichnet, daß die Formaldehydkonzentration während der gesamten Kondensationsreaktion zwischen 1,2 und 6 Gew.-% gehalten wird.

4. Verfahren nach Anspruch 1 bis 3, dadurch gekennzeichnet, daß als Co-Katalysator während der Startphase der Kondensationsreaktion Formose eingesetzt wird.

## Revendications

1. Procédé de production de composés polyhydroxyliques de bas poids moléculaire par condensation d'hydrate de formaldéhyde en présence d'un catalyseur consistant en hydroxyde de calcium ainsi que d'un cocatalyseur consistant en composés aptes à former le groupement endiol, caractérisé en ce qu'il consiste à ajuster à une valeur de 9 à 12, par addition d'hydroxyde de calcium, le pH d'une solution, contenant du formaldéhyde, du cocatalyseur dans l'eau et, le cas échéant, dans des alcools monovalents ou polyvalents de bas poids moléculaire et/ou dans des composés polyhydroxyliques de poids moléculaire élevé, à une température de 80 à 110°C, de manière à déclencher la condensation de l'hydrate de formaldéhyde, puis à ajouter une solution aqueuse de formaline contenant 20 à 65% en poids de formaldéhyde et/ou une dispersion de para-formaldéhyde ainsi que de l'hydroxyde de calcium de manière que le mélange réactionnel soit maintenu à une température de 80 à 110°C et dans une plage de pH de 7,5 à 9,5, la concentration en formaldéhyde pendant toute la durée de la réaction de condensation étant maintenue entre 0,5 et 10% en poids, par rapport au mélange réactionnel total, et finalement, à eliminer éventuellement la quantité restante de 0,5 à 10% en poids de formaldéhyde par poursuite de la condensation à des valeurs de pH inférieures à 7 ou par réaction avec d'autres composés réactifs envers l'hydrate de formaldéhyde.

2. Procédé suivant la revendication 1, caractérisé en ce que le pH est maintenu entre 9 et 10 pendant la phase initiale de la réaction, puis à une valeur comprise entre 8 et 9.

3. Procédé suivant l'une des revendications 1 et 2, caractérisé en ce que la concentration en formaldéhyde pendant toute la durée de la réaction de condensation est maintenue entre 1,2 et 6% en poids.

4. Procédé suivant l'une quelconque des revendications 1 à 3, caractérisé en ce que le formose est utilisé comme cocatalyseur pendant la phase initiale de la réaction de condensation.

## Claims

1. A process for the production of low molecular weight polyhydroxyl compounds by condensing formaldehyde hydrate in the presence of calcium hydroxide as catalyst and in the presence of compounds capable of enediolformation as co-catalyst, characterised in that a formaldehyde-containing solution of the co-catalyst in water and optionally low molecular weight monohydric or polyhydric alcohols and/or relatively high molecular weight polyhydroxyl compounds is adjusted to a pH value of from 9 to 12 by the addition of calcium hydroxide at a temperature of from 80 to 110°C so that condensation of the formaldehyde hydrate is started, after which an aqueous formalin solution

and/or paraformaldehyde dispersion containing from 20 to 65%, by weight, of formaldehyde and calcium hydroxide are added at such a rate that the reaction mixture is maintained at a pH value of from 7.5 to 9.5 et a temperature of from 80 to 110°C, the concentration of formaldehyde being maintained at from 0.5 to 10%, by weight, based on the total reaction mixture, throughout the condensation reaction, and finally the formaldehyde residue, amounting to from 0.5 to 10%, by weight, is optionally removed by further condensation at pH values below 7 or by reaction with other compounds that are reactive to formaldehyde hydrate.

2. A process according to Claim 1, characterised in that a pH value of from 9 to 10 is maintained during the starting phase of the reaction and is subsequently adjusted to from pH 8 to 9.

3. A process according to Claim 1 or 2, characterised in that the concentration of formaldehyde is maintained at from 1.2 to 6% by weight, throughout the condensation reaction.

4. A process according to Claim 1 to 3, characterised in that formose is used as co-catalyst during the starting phase of the condensation reaction.